# FASCICULE DE BREVET EUROPEEN

(11) **EP 3 311 174 B1**
(45) Date de publication et mention de la délivrance du brevet: **28.08.2019**
(21) Numéro de dépôt: 16736402.5
(22) Date de dépôt: 22.06.2016
(51) Int. Cl.: G01N 33/68, C12Q 1/6883

(54) **MÉTHODE DE DIAGNOSTIC DES TROUBLES CAUSÉS PAR L'ALCOOLISATION FOETALE**
VERFAHREN ZUR DIAGNOSE VON FETALER ALKOHOLSPEKTRUMSTÖRUNG
METHOD FOR THE DIAGNOSIS OF FETAL ACOLHOL SPECTRUM DISORDER

(30) Priorité: 22.06.2015 FR 1555727
(43) Date de publication de la demande: 25.04.2018
(73) Titulaire: Centre Hospitalier Universitaire de Rouen, 76000 Rouen (FR); Universite de Rouen Normandie, 76821 Mont Saint Aignan Cedex (FR); Institut National de la Santé et de la Recherche Médicale (INSERM), 75013 Paris (FR)
(72) Inventeur: GONZALEZ, Bruno José, 76150 La Vaupaliere (FR); MARRET, Stéphane, 76000 Rouen (FR); LECUYER, Matthieu Jean Alexandre, 76000 Rouen (FR); LAQUERRIERE, Annie, 76240 Bonsecours (FR); BEKRI, Soumeya, 76160 St Leger du Bourg Denis (FR); LESUEUR, Céline, 76130 Mont Saint Aignan (FR); JEGOU, Sylvie Marguerite Alberte, 76000 Rouen (FR); MARCORELLES, Pascale Yvonne Joséphine, 29200 Brest (FR)
(74) Mandataire: Regimbeau
(86) Numéro de dépôt international: PCT/EP2016/064480
(87) Numéro de publication internationale: WO 2016/207253

(56) Documents cités:
- US-A1- 2010 311 067
- ROSENBERG M J ET AL: "Effects of moderate drinking during pregnancy on placental gene expression", ALCOHOL, PERGAMON PRESS, LONDON, GB, vol. 44, no. 7-8, 1 novembre 2010 (2010-11-01), pages 673-690, XP027527154, ISSN: 0741-8329 [extrait le 2010-01-06]
- HAGHIGHI POODEH S ET AL: "Alcohol-induced premature permeability in mouse placenta-yolk sac barriers in vivo", PLACENTA, vol. 33, no. 10, 2012, pages 866-873, XP028936965, ISSN: 0143-4004, DOI: 10.1016/J.PLACENTA.2012.07.008
- PIIA VUORELA ET AL: "Hepatocyte Growth Factor, Epidermal Growth Factor, and Placenta Growth Factor Concentrations in Peripheral Blood of Pregnant Women With Alcohol Abuse", ALCOHOLISM: CLINICAL AND EXPERIMENTAL RESEARCH., vol. 26, no. 5, 1 mai 2002 (2002-05-01), pages 682-687, XP055261678, US ISSN: 0145-6008, DOI: 10.1111/j.1530-0277.2002.tb02591.x cité dans la demande
- HELSKE S ET AL: "Expression of Vascular Endothelial Growth Factor Receptors 1, 2 and 3 in Placentas From Normal and Complicated Pregnancies", MOLECULAR HUMAN REPRODUCTION, OXFORD UNIVERSITY PRESS, GB - BE, vol. 7, no. 2, 1 janvier 2001 (2001-01-01) , pages 205-210, XP002995374, ISSN: 1360-9947, DOI: 10.1093/MOLEHR/7.2.205
- SYLVIE JÉGOU ET AL: "Prenatal Alcohol Exposure Affects Vasculature Development in the Neonatal Brain", ANNALS OF NEUROLOGY., vol. 72, no. 6, 2012, pages 952-960, XP055261686, BOSTON, US ISSN: 0364-5134, DOI: 10.1002/ana.23699 cité dans la demande

## Description

### INTRODUCTION

L'alcool est un tératogène physique et comportemental. Chez l'Homme, l'exposition prénatale à l'alcool peut conduire à des altérations du développement cérébral. Ainsi, la consommation d'alcool au cours de la grossesse (alcoolisation foetale) constitue la première cause de handicap et notamment de retard mental d'origine non génétique au monde mais également en France.

Les dommages varient selon la période où le foetus a été exposé, les taux d'alcoolémie, les facteurs génétiques et environnementaux, le mode de consommation (chronique, binge drinking).

Le Syndrome d'Alcoolisation Foetale (SAF) constitue la manifestation la plus extrême et invalidante des troubles causés par l'alcoolisation foetale (TCAF). Elle associe des anomalies physiques comme une hypotrophie (retard de croissance), une dysmorphie cranio-faciale et des anomalies neurocomportementales se traduisant par des troubles des fonctions cognitives (troubles de l'attention, de la motricité, de l'apprentissage ou encore de la mémorisation). Le diagnostic des enfants SAF est relativement aisé. Sur la base des anomalies morphologiques, il peut être établi in utero ou à la naissance.

En revanche, de nombreux enfants TCAF ne présentent pas les anomalies morphologiques des enfants SAF, ce qui compromet un diagnostic précoce. Pour autant, ces enfants ne sont pas dépourvus d'atteintes. Ces incapacités/handicaps seront décelé(e)s dans les premières années de vie (hyperactivité, troubles de l'attention) alors que des mois précieux de prise en charge auraient pu être valorisés dès la première année de vie. Ces déficits vont à plus long terme être associés à des inaptitudes sociales, professionnelles et familiales. Le devenir de ces enfants et leur insertion professionnelle sont donc sérieusement hypothéqués. Un diagnostic dès la naissance permettrait une prise en charge précoce de ces enfants essentielle pour réduire au maximum les incapacités associées à l'alcoolisation foetale.

A ce jour des efforts importants ont été entrepris pour identifier des biomarqueurs d'exposition à l'alcoolisation foetale, c'est-à-dire des marqueurs permettant de répondre à la question : l'enfant a-t-il été exposé à l'alcool durant sa vie foetale ?

Or, cette information bien qu'importante n'est pas en mesure d'améliorer à elle seule la prise en charge des nourrissons et pour plusieurs raisons. Tout d'abord, il n'existe pas de seuil de toxicité à l'alcool. Autrement dit une exposition avérée ne sera pas nécessairement associée à des troubles développementaux de l'enfant. En contrepartie, une exposition épisodique à un moment clé du neurodéveloppement ne sera pas pour autant sans conséquences et la notion de fenêtre de vulnérabilité est maintenant clairement admise. En outre, les modes de consommation ont fortement changé. Ainsi, chez les adolescents la consommation épisodique, comme les ivresses associées aux week-ends, est en nette augmentation aussi bien chez les filles que chez les garçons. Enfin, les biomarqueurs d'exposition développés jusqu'alors étant le plus souvent ciblés sur une exposition chronique, il existe un risque réel de faux-négatifs.

US2010/0311067 décrit la détection de biomarqueurs pour détecter une exposition à l'alcool *in utero* et des troubles de l'alcoolisation foetale. Rosenberg *et al.* décrit que l'expression placentaire de, entre autres, Placental Growth Factor, est réduite après consommation modérée d'alcool pendant la grossesse chez les rats (Rosenberg, Alcohol, 44: 673-690, 2010).

Il y a donc un besoin de mettre au point des biomarqueurs qui permettent de suivre les effets de l'alcoolisation in utero.

### DESCRIPTION

La présente invention offre l'opportunité de développer un biomarqueur placentaire d'atteinte cérébrale de l'alcoolisation foetale. Ce type de biomarqueur n'a jamais été développé à ce jour. En effet, les biomarqueurs actuels de l'alcoolisation foetale sont des biomarqueurs dit d'exposition qui permettent de déterminer si la mère a consommé de l'alcool au cours de la grossesse ou si l'enfant a été exposé *in utero.* Mais, hormis les cas les plus sévères (Syndrome d'Alcoolisation Foetale, SAF), un biomarqueur d'exposition ne permet pas de conclure sur un impact cérébral d'une alcoolisation *in utero.* A ce jour, la majorité des enfants TCAF échappe à un diagnostic précoce. Par ailleurs, et pour des raisons économiques évidentes, il n'est pas envisageable de prendre en charge tous les enfants dont la mère aurait consommé de l'alcool pendant la grossesse.

La présente invention permet, contrairement aux biomarqueurs de l'art antérieur, de suivre les effets de l'alcoolisation foetale. En effet, les inventeurs ont montré que le dosage du PlGF permet d'identifier, chez les enfants exposés in utero à l'alcool, ceux qui ont subi des atteintes cérébrales. Notamment, le niveau de PlGF indique quels sont les enfants dont le système vasculaire cérébral est désorganisé, résultant d'une angiogenèse cérébrale altérée. Or ces enfants, à ce jour, échappent à un diagnostic précoce. La présente invention pallie donc au déficit de diagnostic précoce observé pour les enfants TCAF, qui représentent en France 9 cas pour 1000 naissances et dont les signes cliniques (hyperactivité, troubles de l'attention...) ne sont décelés que tardivement (par exemple entre 4 et 5 ans, lors de la scolarisation). La présente invention permet donc de mettre en oeuvre précocement une prise en charge adaptée de ces enfants. Cette prise en charge va notamment consister à stimuler les fonctions motrices, sensorielles et cognitives de l'enfant à une période (petite enfance) où la plasticité cérébrale est maximale.

Dans une étude précédente, (Pia Vuorela et al. Alcoholism : Clinical and Experimental Research., 2002) le taux de PlGF a été mesuré dans le sang périphérique des femmes enceintes consommant de l'alcool et a été comparé à celui des femmes enceintes abstinentes. Selon cette étude, la concentration de PlGF dans le sérum augmente chez les femmes enceintes consommant de l'alcool durant le second et le troisième trimestre de la grossesse par rapport aux femmes abstinentes. Contrairement à ce qui a été décrit par Pia Vuorela et al., les inventeurs de la présente invention ont démontré que la consommation d'alcool pendant la grossesse provoque la diminution de l'expression de PlGF au niveau foetal. La diminution du taux de PlGF est associée à une diminution de l'expression de récepteurs pro-angiogéniques cérébraux du foetus et à une réduction de l'angiogenèse cérébrale foetale. Les effets moléculaires de la réduction du taux PlGF, qui miment ceux de l'alcoolisation foetale, démontrent la validité physiologique de la présente invention.

Selon un premier aspect, l'invention a pour objet une méthode *in vitro* de diagnostic de troubles de l'alcoolisation foetale (TCAF) chez un sujet, ladite méthode comprenant des étapes de :
a) mesure de la quantité de PlGF dans un échantillon biologique et
b) comparaison de la quantité de PIGF de l'étape a) avec une référence, et
c) détermination d'un trouble de l'alcoolisation foetale.

On entend par « PlGF » ou « Placental growth factor » ou « facteur de croissance placentaire » (tous ces termes sont synonymes) une protéine de la famille des facteurs de croissance de l'endothélium vasculaire (VEGF). Plus particulièrement, PlGF au sens de l'invention est une protéine de 149 acides aminés hautement similaire à VEGF-A qui est reconnue par le même récepteur que ce dernier, le VEGF-R1. PlGF est fortement exprimé par le placenta, mais pas par le cerveau foetal. PlGF glycosylé en N-terminus est sécrété et fonctionne en dimère pour stimuler l'angiogénèse. Le terme « PlGF » se réfère notamment à l'ensemble des 4 isoformes PlGF1-4 : PlGF-1 et PlGF-3 sont des isoformes qui ne lient pas l'héparine tandis que PlGF-2 et PlGF-4 contiennent des domaines supplémentaires qui permettent de fixer l'héparine. Encore plus préférentiellement, on entend par PlGF une protéine murine dont la séquence est disponible sous le numéro d'accession NP_001258634 ou une protéine humaine dont la séquence est disponible sous le numéro d'accession NP_001193941.1.

On entend par « Troubles Causés par l'Alcoolisation Foetale (TCAF) » l'ensemble des troubles chez l'enfant résultant de l'exposition à l'alcool durant la gestation. Ce terme comprend entre autre l'ensemble des troubles comportementaux qui vont se révéler progressivement avec l'âge. Les enfants présentant ces troubles sont appelés des « enfants TCAF. » Dans leur version la plus sévère, les TACF correspondent au syndrome d'alcoolisation foetale (SAF). Celui-ci se traduit par une dysmorphie craniofaciale (comprenant des fentes palpébrales raccourcies, un sillon naso-labial lisse, allongé, effacé et une lèvre supérieure mince) ; un retard de croissance non spécifique (taille ou poids ou périmètre crânien) prénatal ou postnatal ou les deux ; et des troubles du développement neurologique s'exprimant parfois par un retard mental et plus souvent par des difficultés d'apprentissage. Les enfants atteint de SAF sont appelés des « enfants SAF. »

Les inventeurs ont montré que l'exposition à l'alcool cause des atteintes vasculaires cérébrales. Par « atteinte vasculaire cérébrale », on entend ici toute altération du système vasculaire cérébral, notamment une altération entrainant un fonctionnement altéré, voire défectueux dudit système. Une atteinte vasculaire cérébrale au sens de l'invention peut notamment être une désorganisation du système vasculaire cérébral. Plus particulièrement, l'alcoolisation foetale induit une orientation aléatoire des vaisseaux cérébraux. Selon un mode de réalisation particulier, le trouble de l'alcoolisation foetale est lié à une atteinte vasculaire cérébrale. Encore plus particulièrement, ledit trouble de l'alcoolisation foetale est lié à une désorganisation du système vasculaire cérébral.

On entend par « sujet » un humain, et de préférence un embryon, un foetus ou un enfant. Un « embryon », tel qu'on l'entend ici, correspond à un ovocyte fécondé de moins de trois mois. Par « foetus », on entend ici un individu pris avant la naissance et dont l'âge gestationnel est compris entre 3 et 9 mois. Après l'accouchement, le sujet devient un enfant. Par « enfant », on entend un individu dont l'âge est inférieur à 3 ans. Sont ainsi compris dans la catégorie des enfants, les nouveau-nés, dont l'âge est compris entre 0 et 1 mois, les nourrissons, qui ont entre 1 mois et 2 ans, et les enfants proprement dits, qui sont âgés d'au moins 2 ans. Un « nouveau-né », comme on l'entend ici, peut aussi bien être né à terme qu'être prématuré.

L'expression « un sujet avec des troubles d'alcoolisation foetale » ou « sujet TCAF » telle qu'utilisée ici se rapporte à un embryon, un foetus ou un sujet, en particulier humain, qui est exposé à l'alcool in utero et qui souffre de troubles d'alcoolisation foetale ou qui est en danger de développer en raison de la consommation maternelle d'alcool une des conditions liées aux troubles d'alcoolisation foetale, y compris les effets décrits ci-dessus. En particulier, un sujet TCAF possède un réseau vasculaire cérébral désorganisé, ladite désorganisation étant notamment liée à une orientation aléatoire des vaisseaux cérébraux.

La méthode de l'invention est particulièrement utile parce qu'elle permet de prédire de façon non invasive les déficits cérébraux. Elle permet en effet de détecter à partir d'un échantillon biologique, notamment un échantillon placentaire, les sujets qui risquent de souffrir de TCAF, ce qui permet de les prendre en charge.

On entend par « échantillon biologique » selon l'invention tout échantillon qui peut être prélevé à partir d'un sujet. Alternativement, l'échantillon biologique est un échantillon provenant du placenta, notamment du cordon ombilical. En effet, le PlGF est exprimé par des cellules du placenta tout au long de la grossesse. Cela permet de doser le PlGF sans attenter à l'intégrité du sujet, en particulier quand celui-ci est un embryon ou un foetus. De façon générale, l'échantillon biologique doit permettre la détermination du niveau d'expression du marqueur biologique utilisé dans l'invention.

L'échantillon à tester peut être utilisé comme obtenu directement à partir de la source biologique ou la suite d'un prétraitement pour modifier le caractère de l'échantillon. Par exemple, un tel prétraitement peut inclure la préparation de plasma à partir de sang, la dilution de fluides visqueux et ainsi de suite. Des procédés de prétraitement peuvent aussi impliquer la filtration, la précipitation, la dilution, la distillation, le mélange, la concentration, l'inactivation de composants perturbateurs, l'addition des réactifs, une lyse, etc. En outre, il peut être bénéfique de modifier un échantillon d'essai solide pour former un milieu liquide ou pour libérer l'analyte.

La protéine PlGF est une protéine sécrétée (DeFalco, Exp Mol Med. 44(1): 1-9, 2012). Les échantillons biologiques préférés pour la détermination du niveau d'expression dudit biomarqueurs comprennent en particulier les échantillons de sang, de plasma, ou de lymphe. De préférence, l'échantillon biologique est un échantillon de sang. De façon encore plus préféré, l'échantillon biologique est un échantillon de sang du placenta ou de sang de cordon. Celui-ci est en effet habituellement recueilli lors de l'accouchement. Le sang des vaisseaux placentaires peut alors être obtenu pour mesurer le niveau de PlGF dans le sang. Cela permet ainsi un diagnostic non invasif d'un trouble de l'alcoolisation foetale, notamment d'une atteinte cérébrale. En effet, le simple dosage du PGlF dans le sang permet de déterminer si l'exposition *in utero* à l'alcool a entrainé des TCAF, notamment à cause d'une désorganisation vasculaire cérébrale.

Les inventeurs ont ainsi montré que le PlGF permet de déterminer qu'une atteinte cérébrale a eu lieu, à la différence des biomarqueurs de l'art antérieur qui ne détectaient que l'exposition du foetus à l'alcool. Le PlGF est donc un biomarqueur fiable des TCAF. Par « biomarqueur », on entend au sens de la présente demande une caractéristique qui est objectivement mesurée et évaluée comme indicateur de processus biologiques normaux, de processus pathogéniques, ou de réponses pharmacologiques à une intervention thérapeutique. Un biomarqueur désigne donc toute une gamme de substances et de paramètre divers. Par exemple, un biomarqueur peut être une substance dont la détection indique un état pathologique particulier (par exemple la présence de la protéine C activée en tant que marqueur d'une infection), ou au contraire une substance dont la détection indique un état physiologique spécifique. Le biomarqueur selon la demande est préférentiellement un gène, les produits d'un gène tels que ses transcrits et les peptides issus de ses transcrits, un lipide, un sucre ou un métabolite.

Selon un aspect de la présente demande, le biomarqueur est un gène, les produits d'un gène tels que des transcrits ou des peptides, un lipide, un sucre ou un métabolite dont les changements d'expression, en particulier de niveau d'expression, corrèlent avec un état physiologique de l'enfant résultant d'une exposition in utero à l'alcool. Selon un aspect particulier, le biomarqueur est un peptide ayant une activité de facteur de croissance.

Le biomarqueur candidat est préférablement un marqueur génique, un marqueur protéique, un marqueur lipidique ou un marqueur métabolique. Pour chacun de ces types de marqueurs, de nombreuses méthodes sont à la disposition de l'homme du métier pour mesurer l'expression dudit biomarqueur et ainsi identifier une différence d'expression entre les enfants exposés *in utero* à l'alcool et les enfants sains, c'est-à-dire n'ayant pas été exposés à l'alcool. Le biomarqueur utilisé dans la méthode de l'invention est le PlGF.

Dans un premier mode de réalisation, ledit marqueur est un marqueur génique ou un marqueur protéique.

Dans ce cas, la méthode de l'invention peut comprendre une ou plusieurs étapes intermédiaires entre le prélèvement de l'échantillon de cellules cutanées et la mesure de l'expression de PGLF, lesdites étapes correspondant à l'extraction à partir dudit échantillon de placenta d'un échantillon d'ARNm (ou de l'ADNc correspondant) ou d'un échantillon de protéine. Celui-ci peut ensuite être directement utilisé pour mesurer l'expression de PGLF. La préparation ou l'extraction d'ARNm (ainsi que la rétrotranscription de celui-ci en ADNc) ou de protéines à partir d'un échantillon cellulaire ne sont que des procédures de routine bien connues de l'homme du métier. Une fois qu'un échantillon d'ARNm (ou d'ADNc correspondant) ou de protéine est obtenu, l'expression de PGLF, au niveau soit des ARNm (c'est-à-dire dans l'ensemble des ARNm ou des ADNc présents dans l'échantillon), soit des protéines (c'est-à-dire dans l'ensemble des protéines présentes dans l'échantillon), peut être mesurée. La méthode utilisée pour ce faire dépend alors du type de transformation (ARNm, ADNc ou protéine) et du type d'échantillon disponible.

Quand l'expression de PGLF est mesurée au niveau de l'ARNm (ou d'ADNc correspondant), n'importe quelle technologie habituellement utilisée par l'homme du métier peut être mise en oeuvre. Ces technologies d'analyse du niveau d'expression des gènes, comme par exemple l'analyse du transcriptome, incluent des méthodes bien connues telles que la PCR (Polymerase Chain Reaction, si on part d'ADN), la RT-PCR (Reverse Transcription-PCR, si on part d'ARN) ou la RT-PCR quantitative ou encore les puces d'acides nucléiques (dont les puces à ADN et les puces à oligonucléotides) pour un plus haut débit.

Par « puces d'acides nucléiques », on entend ici plusieurs sondes d'acides nucléiques différentes qui sont attachées à un substrat, lequel peut être une micropuce, une lame de verre, ou une bille de la taille d'une microsphère. La micropuce peut être constituée de polymères, de plastiques, de résines, de polysaccharides, de silice ou d'un matériau à base de silice, de carbone, de métaux, de verre inorganique, ou de nitrocellulose.

Les sondes peuvent être des acides nucléiques tels que les ADNc (« puce à ADNc »), les ARNm (« puce à ARNm ») ou des oligonucléotides (« puce à oligonucléotides »), lesdits oligonucléotides pouvant typiquement avoir une longueur comprise entre environ 25 et 60 nucléotides.

Pour déterminer le profil d'expression d'un gène particulier, un acide nucléique correspondant à tout ou partie dudit gène est marqué, puis mis en contact avec la puce dans des conditions d'hybridation, conduisant à la formation de complexes entre ledit acide nucléique cible marqué et les sondes attachées à la surface de la puce qui sont complémentaires de cet acide nucléique. La présence de complexes hybridés marqués est ensuite détectée.

Ces technologies permettent de suivre le niveau d'expression d'un gène en particulier ou de plusieurs gènes voire même de tous les gènes du génome (full genome ou full transcriptome) dans un échantillon biologique (cellules, tissus..). Ces technologies sont utilisées en routine par l'homme du métier et il n'est donc pas besoin de les détailler ici. Des exemples de mises en oeuvre de l'invention basées sur l'analyse d'expression génique (puces à ADNc) et sur la PCR quantitative sont décrits dans la section expérimentale.

Alternativement, il est possible d'utiliser toute technologie actuelle ou future permettant de déterminer l'expression des gènes sur la base de la quantité d'ARNm dans l'échantillon. Par exemple, l'homme du métier peut mesurer l'expression d'un gène par hybridation avec une sonde d'acide nucléique marquée, comme par exemple par Northern blot (pour l'ARNm) ou par Southern blot (pour l'ADNc), mais aussi par des techniques telles que la méthode d'analyse sérielle de l'expression des gènes (SAGE) et ses dérivés, tels que LongSAGE, SuperSAGE, DeepSAGE, etc. Il est aussi possible d'utiliser des puces à tissu (aussi connues en tant que TMAs : « tissue microarrays »). Les tests habituellement employés avec les puces à tissu comprennent l'immunohistochimie et l'hybridation fluorescente in situ. Pour l'analyse au niveau de l'ARNm, les puces à tissu peuvent être couplées avec l'hybridation fluorescente in situ. Enfin, il est possible d'utiliser le séquençage massif en parallèle pour obtenir déterminer la quantité d'ARNm dans l'échantillon (RNA-Seq ou « Whole Transcriptome Shotgun Sequencing »). À cet effet, plusieurs méthodes de séquençage massif en parallèle sont disponibles. De telles méthodes sont décrites dans, par exemple, US 4,882,127, U.S. 4,849,077; U.S. 7,556,922; U.S. 6,723,513; WO 03/066896; WO 2007/111924; US 2008/0020392; WO 2006/084132; US 2009/0186349; US 2009/0181860; US 2009/0181385; US 2006/0275782; EP-B1-1141399; Shendure & Ji, Nat Biotechnol., 26(10): 1135-45. 2008; Pihlak et al., Nat Biotechnol., 26(6) : 676- 684, 2008 ; Fuller et al., Nature Biotechnol., 27(11): 1013-1023, 2009; Mardis, Genome Med., 1(4): 40, 2009; Metzker, Nature Rev. Genet., 11(1): 31-46, 2010.

Quand l'expression du marqueur est mesurée au niveau protéique, il est possible d'employer des anticorps spécifiques, en particulier dans des technologies bien connues telles que l'immunoprécipitation, l'immunohistologie, le western blot, le dot blot, l'ELISA ou l'ELISPOT, les tests immunologiques par électrochimiluminescence (ECLIA), les puces à protéines, les puces à anticorps, ou les puces à tissu couplées à l'immunohistochimie. Parmi les autres techniques qui peuvent être utilisées sont comprises les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques voltammétrie et d'ampérometrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, and biréfringence ou index de réfraction (par exemple, par résonance des plasmons de surface, ou « surface plasmon resonance » en anglais, par ellipsometry, par méthode de miroir résonnant, tec.), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS). Toutes ces techniques sont bien connues de l'homme du métier et il n'est pas nécessaire de les détailler ici.

De préférence, l'expression du PlGF est mesurée au niveau protéique. Plus préférablement, l'expression du PlGF est mesurée à l'aide d'un test employant des anticorps spécifiques reconnaissant ledit biomarqueur, en particulier dans des technologies bien connues telles que l'immunoprécipitation, l'immunohistologie, l'électrochemiluminescence (ECLIA), le western blot, le dot blot, l'ELISA ou l'ELISPOT, les puces à protéines, les puces à anticorps, ou les puces à tissu couplées à l'immunohistochimie. Des anticorps dirigés contre PlGF sont disponibles commercialement (voir par exemple, R&D Systems, Danta Cruz, Abcam, etc.) et peuvent être utilisés dans les méthodes de l'invention. Encore plus préférablement, l'expression du PlGF est mesurée par Western Blot ou par ELISA.

Dans un aspect préférentiel de la demande, il peut être utile de comparer le niveau de PlGF obtenu à l'étape a) de la méthode avec un niveau de référence.

Par « un niveau d'expression de référence d'un marqueur biologique », on entend au sens de la présente demande tout niveau d'expression dudit marqueur utilisé à titre de référence. Par exemple, un niveau d'expression de référence peut être obtenu en mesurant le niveau d'expression du marqueur d'intérêt dans un échantillon biologique d'un sujet sain, par exemple un placenta d'un sujet sain, c'est-à-dire un sujet qui n'a pas été exposé à l'alcool *in utero.* Dans ce cas, un niveau de PlGF de l'étape a) inférieur au niveau de référence indique un TCAF. En particulier, les inventeurs ont montré qu'un niveau de PlGF inférieur à celui d'un sujet sain signale une organisation vasculaire cérébrale défectueuse.

Selon un mode avantageux de réalisation de la présente invention, l'expression du marqueur candidat est normalisée par rapport à l'expression d'un marqueur témoin. Un « marqueur témoin » est un marqueur dont l'expression est identique quels que soient le type cellulaire considéré et l'âge du donneur. Selon un mode de réalisation particulier, quand le biomarqueur candidat est un marqueur génique ou un marqueur protéique, le marqueur témoin est un gène qui est exprimé dans tous les types cellulaires, indépendamment de l'âge du sujet, ou son produit protéique. Dans un mode de réalisation plus particulier, ledit marqueur témoin est un gène de ménage ou le produit protéique dudit gène de ménage. Un gène de ménage est un gène qui est exprimé dans tous les types cellulaires et qui fournit une fonction de base qui est nécessaire pour la survie de la cellule. Une liste de gènes de ménage humain peut par exemple être trouvée dans Eisenberg et al. (Trends in Genetics 19: 362-365, 2003). Un gène de ménage préféré pour être utilisé dans l'invention est un gène sélectionné dans le groupe constitué de B2M, TFRC, YWHAZ, RPLO, 18S, GUSB, UBC, TBP, GAPDH, PPIA, POLR2A, ACTB, PGK1, HPRT1, IPO8 et HMBS.

La méthode de l'invention est particulièrement utile parce qu'elle permet d'effectuer un diagnostic non invasif dès un âge précoce. Les enfants qui ont été diagnostiqué comme ayant subi des dommages cérébraux à la suite d'une exposition utérine à l'alcool peuvent ainsi être pris en charge précocement et rapidement. Or il a été montré que plus la prise en charge est précoce et meilleure est la récupération fonctionnelle et cognitive (Toutain et al., Pychotropes, 13 : 49-68, 2007).

Selon un autre aspect, la demande a pour objet une méthode de traitement de troubles de l'alcoolisation foetale chez un sujet. Ladite méthode comprend des étapes de :
a) diagnostic de TCAF chez ledit sujet par l'une quelconque des méthodes ci-dessus ; et
b) traitement dudit sujet si l'étape a) conclut que ledit sujet présente des TCAF.

Par « traitement », on entend ici toute action permettant de diminuer ou d'éradiquer les symptômes ou les cause des TCAF. Un traitement peut comprendre l'administration d'une substance pharmacologique et/ou un suivi psychothérapeutique.

L'invention sera décrite plus précisément au moyen des exemples ci-dessous. Lesdits exemples sont fournis ici à titre d'illustration et ne sont pas, sauf indication contraire, destinés à être limitatifs.

### LÉGENDES DES FIGURES

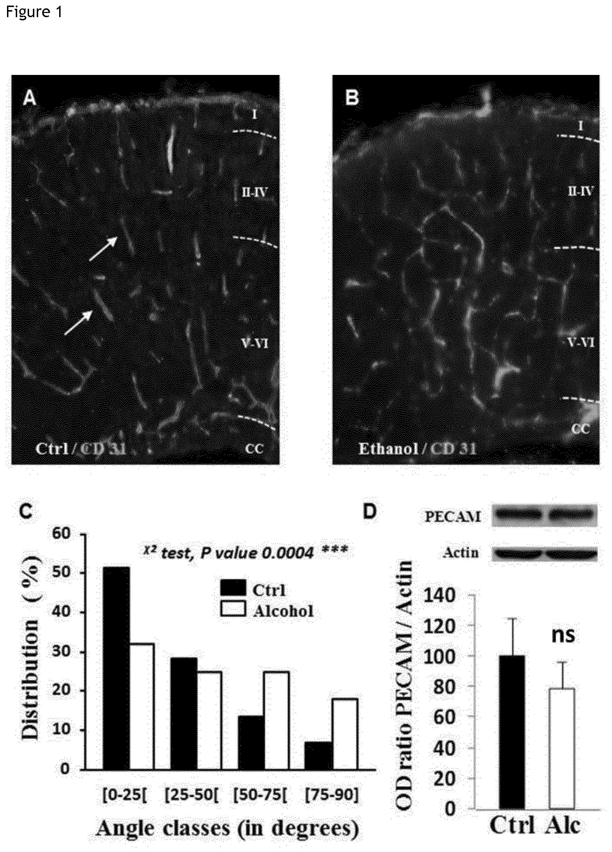

**Figure 1****. Effets de l'exposition alcoolique *in utero* sur l'angiogénèse corticale chez les embryons E20 de Souris. A,B:** Effets de l'exposition alcoolique foetale de GD15 à GD20 sur l'organisation des microvaisseaux corticaux chez des animaux témoin (A) et exposés à l'alcool (B). Les microvaisseaux du cerveau ont été visualisés par immunohistochimie contre CD31. Les flèches indiquent les microvaisseaux du cerveau présentant une orientation radiale dans le groupe « Témoin ». Il faut noter une perte de l'organisation radiale dans le groupe « Alcool ». I-VI: Couches corticales; CC: Corpus callosum. **C:** Distribution de l'orientation (catégories d'angles) des microvaisseaux corticaux dans le cortex immature de foetus GD20. L'analyse statistique a été réalisée à l'aide du test x². **D:** Quantification par western blot des effets de l'exposition alcoolique foetale durant la dernière semaine de gestation sur l'expression corticale de CD31 à GD20. ns vs le groupe « Témoin » à l'aide d'un test t non-apparié.

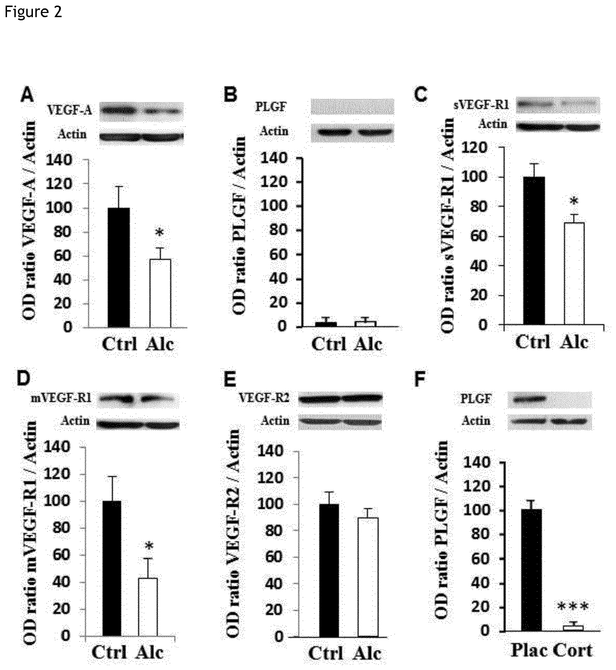

**Figure 2****. Effets de l'exposition alcoolique *in utero* sur l'expression des membres de la famille VEGF/PIGF chez les embryons E20 de Souris. A-E:** Quantification par western blot des niveaux protéiques de VEGFA (A), PlGF (B), sVEGF-R1 (C), mVEGF-R1 (D) et VEGF-R2 dans le cortex des groupes « Témoin » et « Alcool ». **F:** Comparaison par western blot des niveaux protéiques de PlGF dans le cortex et le placenta des embryons E20 du groupe « Témoin ». ***p<0.001 vs le groupe « Témoin » à l'aide d'un test t non-apparié.

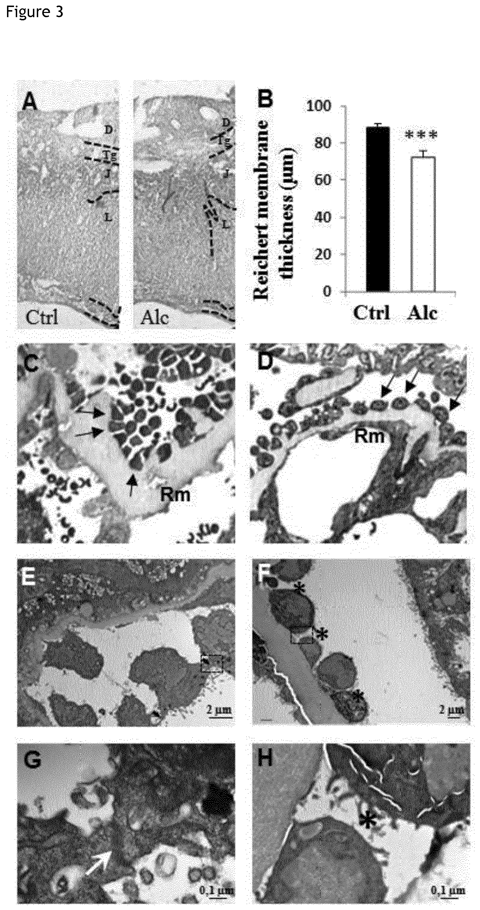

**Figure 3****. Effets de l'exposition alcoolique *in utero* sur les caractéristiques ultrastructurelles du placenta chez les souris GD20. A:** Observation par marquage au violet de Cresyl de l'effet de l'exposition alcoolique sur la structure laminaire du placenta. Le côté maternel du placenta est orienté vers le haut. L'alcool affecte la ségrégation des zones de jonction et du labyrinthe (lignes pointillées). **B:** Quantification par analyse d'images des effets de l'alcool sur l'épaisseur de la membrane de Reichert. **C,D:** Observation à faible grossissement de la couche des trophoblastes géants dans les groupes « Témoin » (C) et « Alcool » (D). Les flèches indiquent les trophoblastes géants. Ceux-ci ont une forme rectangulaire typique dans le placenta du groupe « Témoin », alors que dans le groupe « Alcool », ils ont une forme arrondie. **E-H:** Images acquises par microscopie électronique à grossissement moyen (E,F) et fort (G,H) montrant la morphologie cellulaire des trophoblastes géants et la présence de *zonula occludens* (flèches) dans les groupes « Témoin » (E,G) et « Alcool » (F,H). La *zonula occludens* (étoiles) n'est plus visible dans les animaux traités à l'alcool. Les inserts dans E et F indiquent la zone observée à plus fort grossissement en G et H, respectivement. D: maternal decidua; J: zone de jonction; L: zone du labyrinthe; Tg: couche de trophoblastes géants. ***p<0.001 vs le groupe « Témoin » à l'aide d'un test t non-apparié.

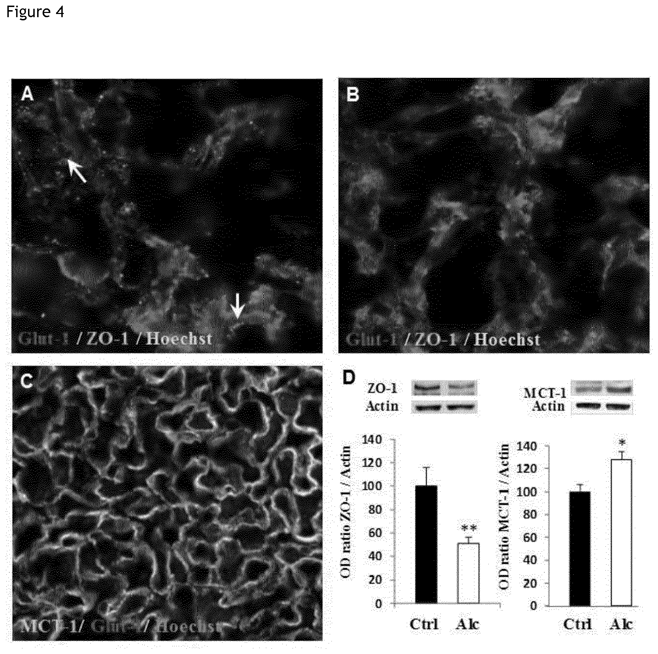

**Figure 4****. Effets de l'exposition alcoolique *in utero* sur l'expression des protéines participant à la barrière placentaire et au métabolisme énergétique placentaire. A,B:** Observation par immunohistochimie de la protéine ZO-1 dans la zone du labyrinthe du placenta de souris des groupes « Témoin » (A) et « Alcool » (B). La protéine ZO-1 apparait comme formant des groupes de points (flèches) dans le groupe « Témoin » tandis que le marquage est diffus dans le groupe « Alcool ». Les couches de trophoblastes ont été mises en évidence par immunoréactivité avec le transporteur de glucose Glut-1. Les noyaux ont été marqués au Hoechst. **C:** Double marquage avec des anticorps contre les transporteurs de monocarboxylate MCT-1 et de glucose dans la zone du labyrinthe d'un placenta « Témoin ». Par contraste avec Glut-1, l'expression de MCT-1 est associée avec la couche maternelle du syncytiotrophoblaste. Les noyaux ont été marqués au Hoechst. **D:** Quantification par western blot des niveaux d'expression des protéines ZO-1 et MCT-1 dans les placentas des groupes « Témoin » et « Alcool ». *p<0.05, **p<0.01 vs le groupe « Témoin » à l'aide d'un test t non-apparié.

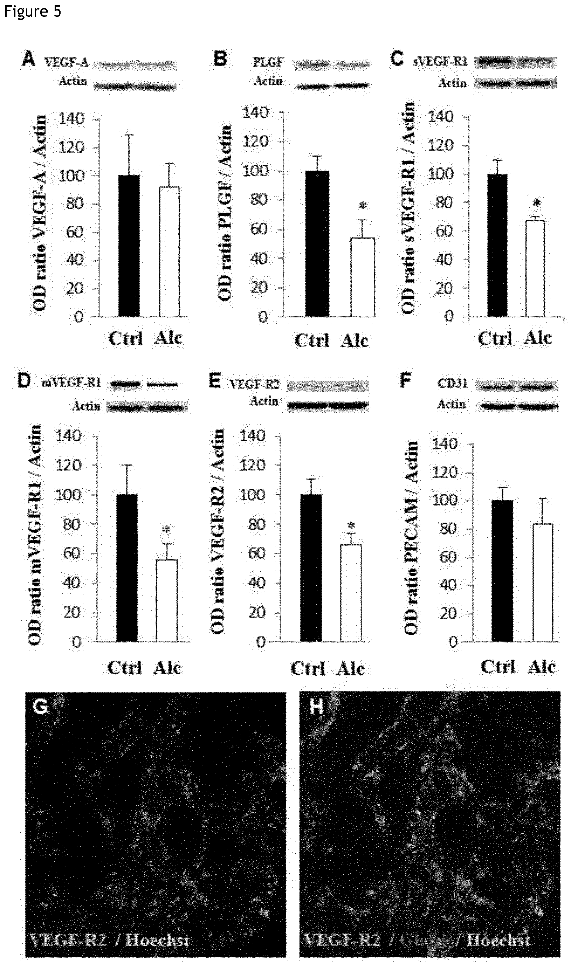

**Figure 5****. Effets de l'exposition alcoolique *in utero* sur l'expression des membres de la famille VEGF/PIGF dans des placentas murins. A-F:** Quantification par western blot des effets de l'exposition à l'alcool pendant la dernière semaine de gestation sur l'expression placentaire of VEGF-A (A), PlGF (B), sVEGF-R1 (C), mVEGF-R1 (D), VEGF-R2 (E) et CD31 (F) à GD20. **G,H:** Marquage par immunohistochimie montrant la distribution de VEGF-R2 (G) dans les couches du syncytiotrophoblaste du placenta marquées avec Glut-1 (H). Les noyaux ont été marqués au Hoechst. *p<0.05 vs le groupe « Témoin » à l'aide d'un test t non-apparié.

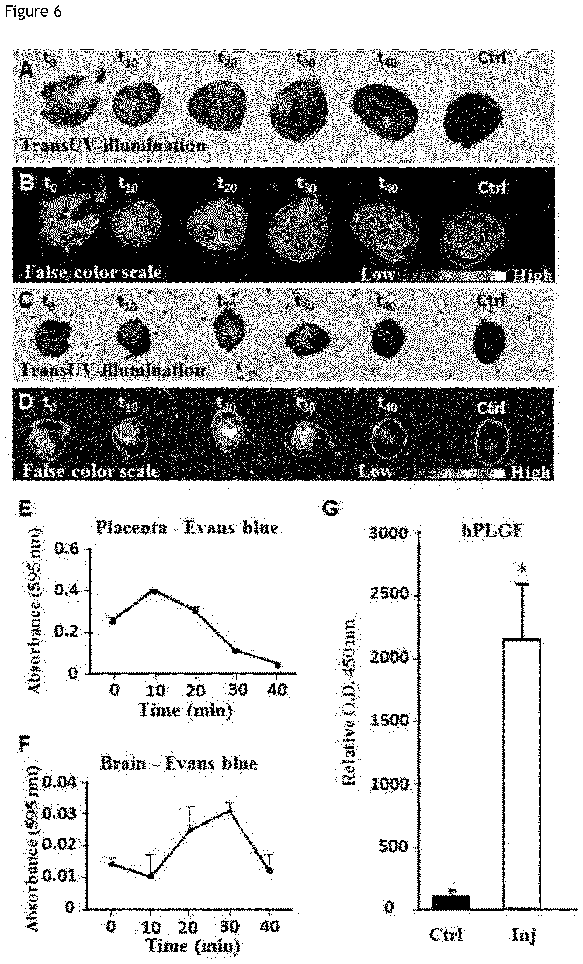

**Figure 6****. Diffusion du Bleu Evans injecté *in utero* du placenta dans le cerveau du foetus. A,B:** Visualisation au cours du temps du Bleu Evans administré par microinjection dans le placenta d'une souris gravide à GD15. La fluorescence a été détectée par illumination aux UV (A) et est représentée à l'aide d'une échelle de couleurs factices (B). **C,D:** Visualisation au cours du temps de la fluorescence du Bleu Evans dans le cerveau des foetus après une microinjection placentaire à GD15. La fluorescence a été détectée par illumination aux UV (C) et est représentée à l'aide d'une échelle de couleurs factices (D). **E,F:** Quantification au cours du temps par spectrophotométrie de l'absorbance à 595 nm du signal du Bleu Evans injecté dans les placentas (E) et de la suite dans les cerveaux des foetus correspondants (F). **G:** Quantification par ELISA du PlGF humain dans le cerveau de foetus de souris 30 min après injection du hPlGF dans les placentas des souris gravides à GD15. *p<0.05 vs le groupe « Témoin » à l'aide d'un test t non-apparié.

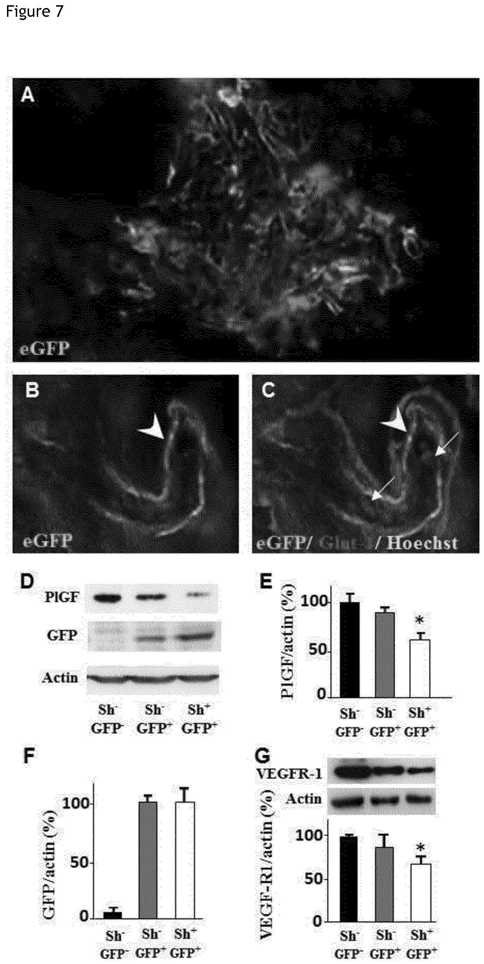

**Figure 7** **: Effet de la répression du PIGF placentaire par transfection *in utero* sur les niveaux de VEGF-R1 du cerveau. A:** Microphotographie montrant l'expression de la eGFP 48 heures après la transfection *in utero* d'un plasmide codant la eGFP dans des placentas de souris gravides à GD15. **B,C:** Triple marquage eGFP/Glut-1/Hoechst montrant que la fluorescence de la eGFP (B) est essentiellement associée avec la couche trophoblastique foetale marquée avec Glut-1 (C; têtes de flèches). La couche trophoblastique maternelle qui est aussi marquée par Glut-1 n'est pas transfectée. La couche trophoblastique foetale est identifiée par la présence de globules rouges nucléés caractéristiques de la circulation foetale (flèche). D: Visualisation par western blot des protéines PlGF, GFP et actine dans les placentas d'animaux non-transfectés (sh⁻/GFP⁻), transfectés par la GFP (sh⁻/GFP⁺) et transfectés par shPlGF/GFP (sh⁺/GFP⁺). **E,F:** Quantification par western blot des niveaux d'expression de PlGF (E) et de GFP (F) dans les placentas d'animaux non-transfectés (sh⁻/GFP⁻), transfectés par la GFP (sh⁻/GFP⁺) et transfectés par shPlGF/GFP (sh⁺/GFP⁺). **G:** Quantification par western blot des niveaux d'expression de VEGF-R1 dans le cerveau de foetus provenant de placentas non-transfectés (sh⁻/GFP⁻), transfectés par la GFP (sh⁻/GFP⁺) et transfectés par shPlGF/GFP (sh⁺/GFP⁺). *p<0.05 vs le groupe "sh⁻/GFP⁻" à l'aide du test ANOVA suivi d'un test de comparaisons multiples HSD de Tukey.

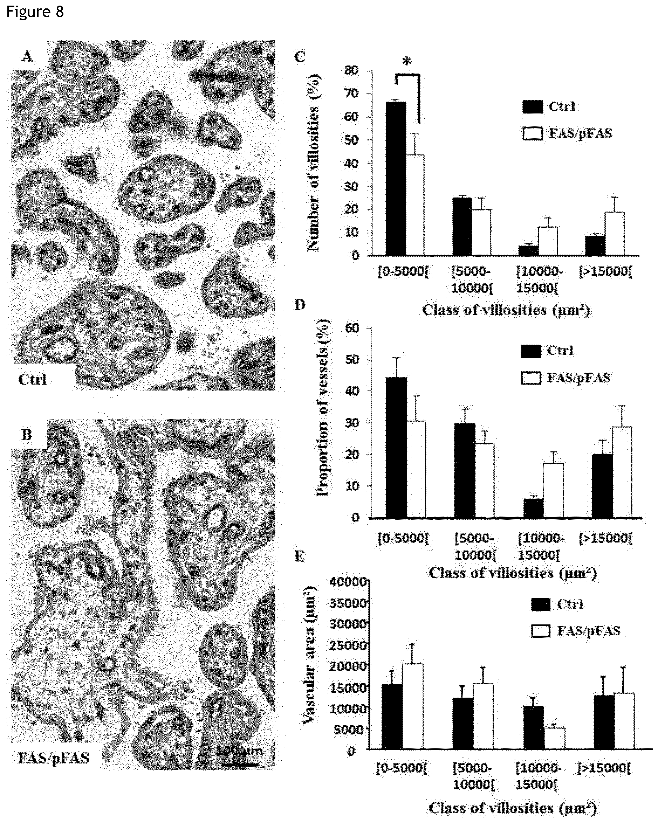

**Figure 8****. Caractérisation morphométrique des effets de l'exposition alcoolique *in utero* sur le placenta humain des semaines gestationnelles 20 à 25. A,B:** Marquage immunohistochimique réalisé contre CD31 et contre-coloration au bleu de toluidine pour visualiser les microvaisseaux (marrons) présents dans les villosités placentaires (bleu) des groupes « Témoin » (A) et "FAS/pFAS" (B) à des âges de gestation [20-25 WG[. **C:** Pourcentage de villosités classées par taille dans les placentas des groupes « Témoin » et "FAS/pFAS" à des âges de gestation [20-25 WG[. **D:** Répartition des vaisseaux par taille de villosités dans les placentas des groupes « Témoin » et "FAS/pFAS" à des âges de gestation [20-25 WG[. **E:** Surface vasculaire par taille de villosités dans les placentas des groupes « Témoin » et "FAS/pFAS" à des âges de gestation [20-25 WG[.*p<0.05 vs le groupe « Témoin » à l'aide d'un test t non-apparié.

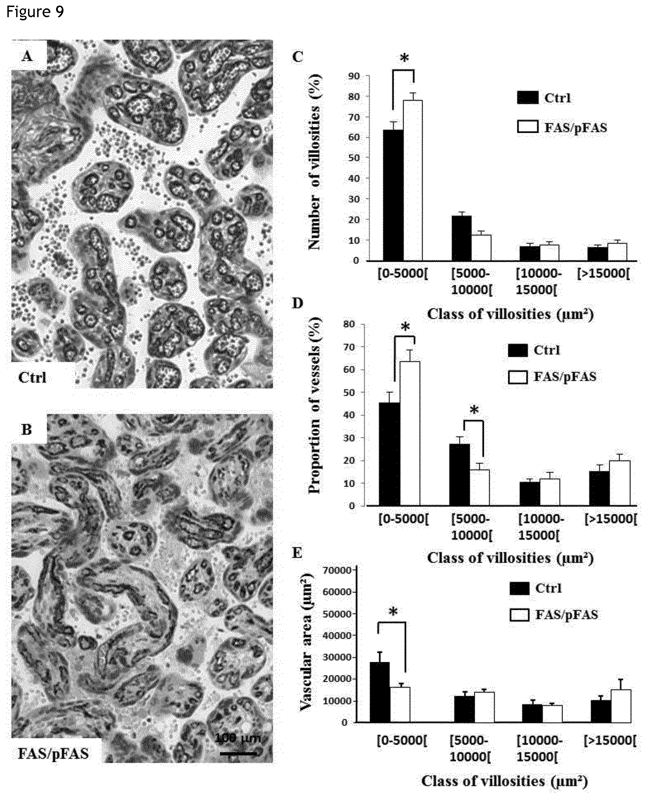

**Figure 9****. Caractérisation morphométrique des effets de l'exposition alcoolique *in utero* sur le placenta humain des semaines gestationnelles 25 à 35. A,B:** Marquage immunohistochimique réalisé contre CD31 et marquage au bleu de toluidine pour visualiser les microvaisseaux (marrons) présents dans les villosités placentaires (bleu) des groupes « Témoin » (A) et "FAS/pFAS" (B) à des âges de gestation [25-35 WG[. **C:** Pourcentage de villosités classées par taille dans les placentas des groupes « Témoin » et "FAS/pFAS" à des âges de gestation [25-35 WG[. **D:** Répartition des vaisseaux par taille de villosités dans les placentas des groupes « Témoin » et "FAS/pFAS" à des âges de gestation [25-35 WG[. **E:** Surface vasculaire par taille de villosités dans les placentas des groupes « Témoin » et "FAS/pFAS" à des âges de gestation [25-35 WG[. *p<0.05 vs le groupe « Témoin » à l'aide d'un test t non-apparié.

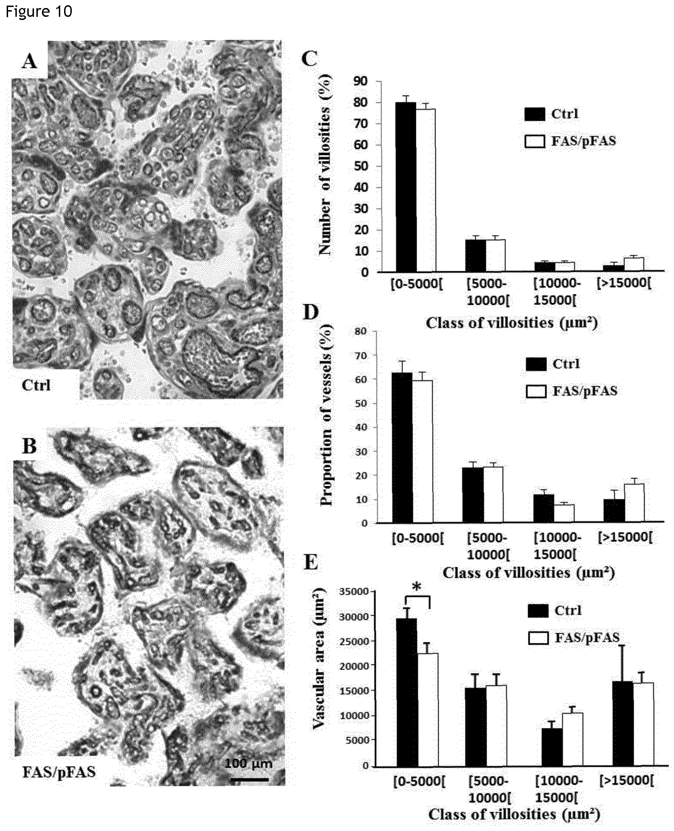

**Figure 10****. Caractérisation morphométrique des effets de l'exposition alcoolique *in utero* sur le placenta humain des semaines gestationnelles 35 à 42. A,B:** Marquage immunohistochimique réalisé contre CD31 et marquage au bleu de toluidine pour visualiser les microvaisseaux (marrons) présents dans les villosités placentaires (bleu) des groupes « Témoin » (A) et "FAS/pFAS" (B) à des âges de gestation allant de [35-42 WG[. La région luminale des microvaisseaux est fortement réduite dans le groupe "FAS/pFas". **C:** Pourcentage de villosités classées par taille dans les placentas des groupes « Témoin » et "FAS/pFAS" à des âges de gestation allant de [35-42 WG[. **D:** Répartition des vaisseaux par taille de villosités dans les placentas des groupes « Témoin » et "FAS/pFAS" à des âges de gestation allant de [35-42 WG[. **E:** Surface vasculaire par taille de villosités dans les placentas des groupes « Témoin » et "FAS/pFAS" à des âges de gestation allant de [35-42 WG[.*p<0.05 vs le groupe « Témoin » à l'aide d'un test t non-apparié.

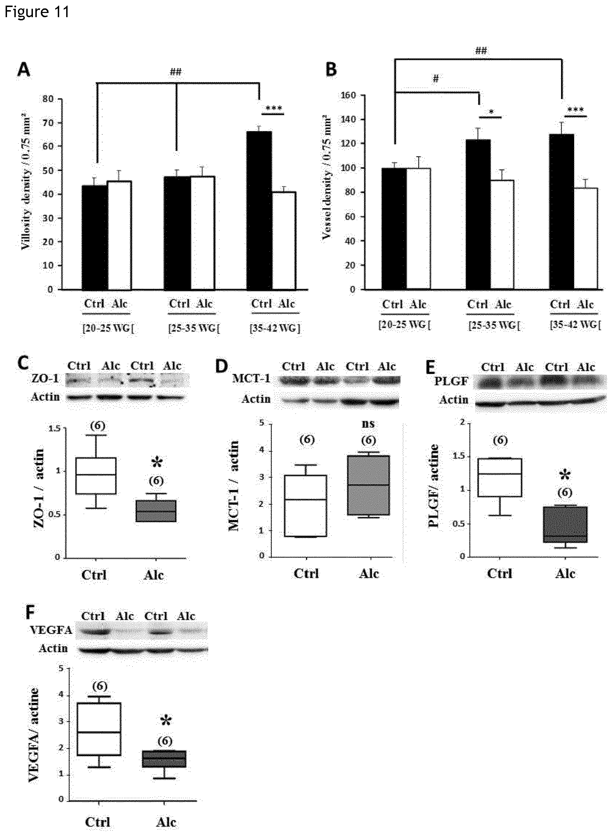

**Figure 11****. Effets au cours du temps de l'exposition alcoolique *in utero* sur les densités de villosités et de vaisseaux dans des placentas humains et caractérisation par western blot de protéines pro-angiogéniques et du métabolisme énergétique. A:** Evolution des densités de villosités dans les placentas des groupes « Témoin » (A) et "FAS/pFAS" (B) à des âges de gestation [20-25 WG[, [25-35 WG[ et [35-42 WG[. **B:** Evolution des densités de vaisseaux dans les placentas des groupes « Témoin » et "FAS/pFAS" à des âges de gestation [20-25 WG[, [25-35 WG[et [35-42 WG[. ^{#}p<0.05, ^{##}p<0.01 vs le groupe « Témoin » comme indiqué sur le graphe. *p<0.05, ***p<0.001 pour les groupes « Témoin » vs « Alcool » pour une classe donnée d'âge gestationnel. **C-H:** Quantification par western blot des niveaux protéiques de ZO-1 (C), MCT-1 (D), PlGF (E), VEGFA (F), VEGF-R1 (G) and VEGF-R2 (H) dans les placentas des groupes « Témoin » et "FAS/pFAS". *p<0.05 vs le groupe « Témoin » à l'aide d'un test t non-apparié.

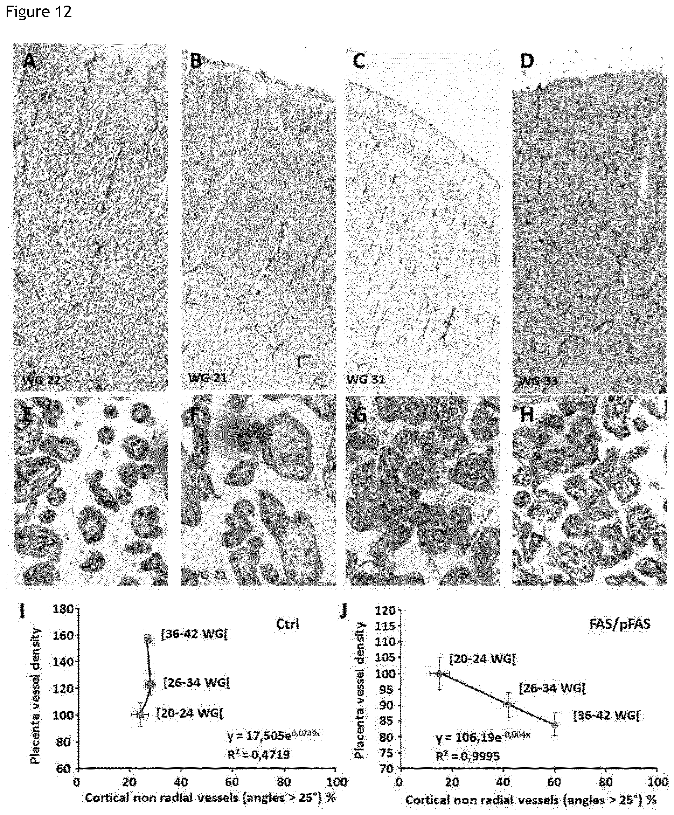

**Figure 12****. Comparaison des atteintes cérébrales et placentaires observées chez les foetus humains et induites par l'exposition alcoolique *in utero* et corrélation statistique. A-H:** Organisation vasculaire dans les cerveaux (A,D) et les placentas (E,H) de patients du groupe « Témoin » a WG22 (A,E) et WG31 (C,G) et organisation vasculaire dans les cerveaux (B,D) et les placentas (F,H) des patients du groupe "FAS/pFAS" à WG21 (B,F) et WG33 (D,H). **I,J:** Corrélation statistique entre la désorganisation vasculaire corticale et la densité vasculaire placentaire dans les patients des groupes « Témoin » (I) et FAS/pFAS (J).

### EXEMPLES

### Anomalies de l'angiogenèse cérébrale suite à une alcoolisation in utero

### Effets d'une exposition in utero à l'alcool sur le développement du réseau vasculaire cérébral

Les présents inventeurs ont précédemment démontré **qu'une alcoolisation prénatale à l'alcool induit une désorganisation vasculaire cérébrale.** En particulier, l'effet de l'alcool est associé à une diminution significative du nombre de vaisseaux corticaux présentant une orientation radiale au profit du nombre de microvaisseaux possédant une orientation aléatoire (Figure 1). Parallèlement à l'étude effectuée chez la Souris, une analyse du système microvasculaire cérébral chez l'Homme a montré que, comme chez la Souris, les microvaisseaux corticaux qui ont une orientation radiale dans le groupe « Témoin » sont totalement désorganisés dans le groupe « FAS/pFAS » (Figure 12 et Jegou et al., 2012).

### Effets d'une exposition in utero à l'alcool sur l'expression de gènes représentatifs du système vasculaire chez la Souris

Les études de RT-PCR quantitative (ARNm) et de Western blot (protéine) ont révélé une dérégulation marquée des taux de récepteurs VEGF-R1 et VEGF-R2 qui relaient les effets pro-angiogéniques de facteurs comme le VEGFA ou encore le PlGF. Les anomalies du réseau vasculaire cérébral sont donc associées à une dérégulation de l'expression de récepteurs pro-angiogéniques cérébraux (Figure 2 et Jegou et al., 2012).

### Anomalies de l'angiogenèse placentaire suite à une alcoolisation in utero

Différents paramètres placentaires ont été étudiés chez la Souris (Figures 3-5) et chez l'Homme (Figures 8-10) par une approche immunohistochimique couplée à une analyse morphométrique comprenant notamment la densité et la taille des villosités placentaires, la densité et la surface vasculaire ou encore la proportion de vaisseaux par villosité. Chez l'Homme, ces paramètres ont été mesurés et comparés entre 34 placentas d'individus témoins et 36 placentas provenant d'individus exposés *in utero* à l'alcool. Les placentas ont été répartis en trois classes d'âges comparables à celles de l'étude cérébrale (Jegou et al., 2012). Sont présentés dans ce document les résultats concernant les classe d'âge [20-25GW[, [25-35GW[et [35-42GW[.

En particulier, l'analyse morphométrique indique que la répartition des vaisseaux placentaires par tailles de villosité et la surface vasculaire sont significativement impactées par l'alcoolisation (Figure 11). De plus, une analyse longitudinale de la densité vasculaire prenant en compte le facteur « âge » indique que dans le groupe « Témoins » l'angiogenèse placentaire augmente fortement entre les classes d'âge [20-25GW[et [25-35GW[. Cette forte vascularisation placentaire s'explique par un développement important du cerveau au cours du troisième trimestre de la grossesse qui nécessite des besoins accrus en oxygène et en nutriments. En revanche, **l'alcoolisation foetale induit une stagnation voire une baisse de la densité vasculaire placentaire** (Figure 11).

En conclusion, les présents résultats indiquent **qu'il existe dans le placenta humain comme dans le cortex cérébral des anomalies vasculaires chez les sujets ayant été exposés à l'alcool.** Ces résultats confortent donc l'hypothèse d'un corrélat entre troubles cérébraux et déficits placentaires de l'angiogenèse.

### Démonstration d'une corrélation entre les anomalies vasculaires placentaires et cérébrales

Les anomalies vasculaires placentaires et cérébrales observées chez l'Homme suite à une alcoolisation *in utero* peuvent être le fruit de processus totalement indépendants sans lien de cause à effet ou, au contraire, étroitement intriqués. Le fait que la source de PlGF soit unique et d'origine placentaire plaide en faveur de la seconde hypothèse. Toutefois, afin de démontrer un lien entre les atteintes vasculaires cérébrales et placentaires, nous avons effectué une étude de corrélation d'une part chez les sujets du groupe « Témoin » et, d'autre part, chez les individus du groupe « FAS/pFAS » (Figure 12).

Les résultats démontrent que dans le groupe « Témoin », l'accroissement de la vascularisation placentaire n'impacte pas l'organisation radiale des vaisseaux corticaux (R² 0,4719). En revanche, le défaut de vascularisation placentaire observé dans le groupe « FAS/pFAS » est étroitement corrélé avec l'orientation aléatoire des vaisseaux corticaux (R² 0,9995). Il existe donc une **interaction très significative entre les altérations vasculaires placentaires et cérébrales.**

### Démonstration d'un lien fonctionnel entre le PlGF placentaire et son récepteur cérébral

L'administration in utero d'une molécule fluorescente au niveau placentaire chez la Souris gestante (GD15) est retrouvée après 20-30 min dans le cerveau du foetus (Figure 6). De plus, du PlGF recombinant humain injecté chez la Souris au niveau du placenta est détecté après 30 min par ELISA au niveau du cerveau foetal (Figure 6). Ces données indiquent que des molécules placentaires et notamment le PlGF sont en mesure d'atteindre le cerveau du foetus.

L'invalidation par transfection in utero placentaire du PlGF murin par des shRNA se traduit par une repression des taux protéiques de PlGF placentaire après 48 heures (Figure 7). Cet effet est associé au niveau cérébral par une chute des niveaux protéiques du récepteur VEGF-R1 (Figure 7). Ces résultats indiquent que i) la répression spécifique du PlGF placentaire impacte directement l'expression du récepteur cérébral, ii) la répression spécifique du PlGF placentaire mime les effets de l'alcool sur l'expression du VEGF-R1 cérébral (Figures 2 et 7).

### Identification des facteurs placentaires biomarqueurs d'une atteinte cérébrale

L'étude de corrélation ci-dessus démontre pour la première fois que les atteintes vasculaires placentaires induites par l'alcoolisation foetale sont en lien direct avec les altérations vasculaires cérébrales. Par conséquent, des **facteurs placentaires** dont le rôle sur l'angiogenèse est avéré deviennent des candidats **biomarqueurs d'atteintes vasculaires cérébrales.**

Les niveaux d'expression de protéines connues pour être soit des acteurs de l'angiogenèse soit des protéines spécifiques du système vasculaire ont été quantifié par western blot. Ce travail a été effectué chez l'animal (Souris ; placenta/cerveau) et chez l'Homme (placenta).

Chez la Souris, la quantification des taux d'expression de VEGF_{A} et de PlGF placentaires démontrent une diminution significative uniquement du PlGF (dont le placenta est la seule source dans l'organisme ; Figure 5). Parallèlement, la quantification des récepteurs au VEGF_{A} et au PlGF indique que l'expression du VEGFR1 (unique récepteur du PlGF) est diminuée aussi bien dans le placenta que dans le cerveau (Figures 2 et 5). Cette diminution, très marquée, est de l'ordre de 50%. L'expression de VEGFR2 au niveau cérébral n'est quant à elle pas affectée. De plus, la quantification de la protéine vasculaire ZO-1, impliquée dans l'établissement de la barrière placentaire et hématoencéphalique est fortement diminuée dans le placenta (Figure 4).

Parallèlement aux travaux menés chez la Souris, l'analyse d'expression protéique a été menée sur des placentas humains dont l'alcoolisation maternelle était avérée et les enfants vivants. Nous avons recueilli 7 placentas « Témoins » et 6 placentas « Alcool » et quantifié par western blot les marqueurs candidats identifiés chez la Souris. Les résultats indiquent que dans le groupe « Alcool » les expressions du PlGF et de ZO-1 sont très fortement diminuées comme chez la Souris (Figure 11). Ces données indiquent que les effets de l'alcoolisation foetale observés aux niveaux placentaire et cérébral sont retrouvés chez deux espèces différentes, la Souris et l'Homme.

### Évaluation des concentrations de PlGF dans le sang du cordon ombilical, le placenta et le sang maternel à partir de deux groupes de patients (contrôle vs in utero exposés à l'alcool)

L'objectif principal de cette étude clinique est de comparer les concentrations de PLGF dans le cordon ombilical et le placenta entre deux groupes de patients et d'effectuer un suivi à 2 et 6 ans du neurodéveloppement des deux groupes de patients. Dans le premier groupe, les patients ont été exposés à l'alcool *in utero.* Le second groupe est un groupe témoin constitué de patients qui n'ont pas été exposés à l'alcool *in utero.*

Cette étude clinique a notamment les objectif suivants :
- comparaison des concentrations de PLGF dans le sang maternel ;
- examen clinique neurologique à la naissance de l'enfant ;
- suivi à l'âge de 2 ans en consultation de pédiatrie pour évaluation du neuro développement, notamment via un questionnaire ASQ (Ages and Stages Questionnaire), et
- suivi à l'âge de 6 ans en consultation pédiatrique pour évaluation du neurodéveloppement et via un questionnaire parental et bilan neuropsychologique.

Dans cette étude clinique 30 femmes ayant consommé de l'alcool pendant leur grossesse et 30 femmes enceintes abstinentes (groupe témoin) sont suivies. Toutes les femmes suivies sont âgées de plus de 18 ans et ont signé un protocole de consentement.

La consommation documentée d'alcool pendant la grossesse est une consommation chronique d'au moins 30g d'alcool par semaine ou consommation aiguë de type « binge drinking » pendant la grossesse (sachant qu' une unité de 10 gr d'alcool pur correspond à 25 cl de bière 4°5, 10 cl de vin à 12°, 3 cl de whisky, 7 cl de Porto...).

Dans le groupe témoin, aucune consommation d'alcool pendant la grossesse n'est documentée.

30 patients dans chaque groupe sont nécessaires pour mettre en évidence une différence de taux de PlGF de 4.7 pg/ dL avec une puissance de test de 80 %

Le dosage du PLGF dans le sang du cordon ombilical et le placenta est effectué par un dosage immunologique par électrochimiluminescence (robot d'analyse par ECLIA du PLGF (Cobas e411 Analyzer) mise à disposition par la société Roche Diagnostic) sur des prélèvements de sang de cordon et de placentas (groupe contrôle vs groupe exposé à l'alcool).

Des prélèvements tissulaires de sang de cordon et de placentas sont effectués puis congelés et conservés à -80°C. Une quantification sur extraits tissulaires du PLGF sanguin et placentaire est ensuite réalisée.

Un examen clinique à la visite de sortie de maternité (poids, taille, périmètre crânien, tonus axial et périphérique, réactivité, reflexes archaïques, adaptations posturales, dysmorphie faciale évocatrice de SAF, malformations éventuelles) est réalisé.

Un suivi des enfants à l'âge de 2 et 6 ans est effectué en ciblant le développement cognitif et les troubles comportementaux.

Lors de la visite à l'âge de 2 ans, le poids, la taille et le périmètre crânien (PC) sont mesurés. Un questionnaire ASQ est rempli et un examen neurologique (IRM cérébrale à la recherche de malformations cérébrales) et des recherches de signes de dysmorphie faciale sont effectués. Une recherche d'une rigidité vasculaire des vaisseaux rétiniens par un ophtalmologiste est également effectuée.

Lors de la visite de l'âge de 6 ans, le poids, la taille et le PC sont mesurés et un examen neurologique et neuropsychologique à l'aide des échelles de neurodéveloppement (WISC IV et NEPSY) sont réalisés. Des questionnaires de Conners destinés aux parents et aux instituteurs (pour le dépistage de l'hyperactivité) et des questionnaires SCQ destiné aux parents (en lien avec le comportement) sont également remplis lors de cette visite.

Dans les deux groupes, à la naissance, à l'âge de 2 ans et à 6 ans, des examens cliniques (comportement, poursuite oculaire-fixation, tonus axial et périphérique, bilan neuro-moteur, réflexes ostéo-tendineux, examen physique complet à la recherche de malformations) et des examens paracliniques (Fond d'oeil, IRM cérébrale, questionnaire parental ASQ, échelles de développement WISC IV et Nepsy, questionnaires parentaux et enseignant de Conners et SCQ) sont réalisés.
La comparaison des 2 groupes de patients est réalisée par le test non paramétrique de Mann Whitney. Un seuil de significativité de 5 % est fixé.

Les résultats obtenus sont conformes à ce qui était attendu.

### Conclusion

Au vu des différents résultats obtenus par les inventeurs chez la Souris et l'Homme, il apparaît que
*i)* l'alcoolisation foetale impacte l'angiogenèse cérébrale et l'organisation du réseau vasculaire cérébral,
*ii)* ces altérations cérébrales sont corrélées avec des anomalies vasculaires du placenta,
*iii)* un facteur pro-angiogénique placentaire est en mesure d'atteindre le cerveau foetal,
*iv)* les anomalies neurodéveloppementales de l'angiogenèse cérébrale chez les enfants TCAF sont associées à une dérégulation du système PlGF placentaire/VEGF-R1 cérébral,
*v)* une invalidation placentaire du PlGF reproduit les effets de l'alcoolisation foetale sur le VEGF-R1 cérébral,
*vi)* une dérégulation des taux placentaires de PlGF suite à une alcoolisation foetale permet de prédire une atteinte cérébrale,
*vii)* un facteur protéique placentaire, le PlGF a été identifié comme un biomarqueur d'atteinte cérébrale induite par une alcoolisation in utero.

## Revendications

1. Méthode *in vitro* de diagnostic de trouble de l'alcoolisation foetale (TCAF) chez un sujet comprenant des étapes de :
a) mesure de la quantité de Placental growth factor (PlGF) dans un échantillon biologique dudit sujet et ;
b) comparaison de la quantité de PlGF de l'étape a) avec une référence, et
c) détermination d'un TCAF chez ledit sujet.

2. La méthode de la revendication 1, **caractérisée en ce que** la référence est une mesure de la quantité de PlGF dans un individu sain.

3. La méthode de l'une quelconque des revendications 1 ou 2, **caractérisée en ce qu'**une quantité de PlGF de l'étape a) inférieure à la référence indique que le sujet souffre de TCAF.

4. La méthode de l'une quelconque des revendications 1 à 3, **caractérisée en ce qu'**une quantité de PlGF de l'étape a) inférieure à la référence indique une désorganisation vasculaire cérébrale chez le sujet.

5. La méthode de l'une quelconque des revendications 1 à 4, **caractérisée en ce que** ledit échantillon biologique provient du placenta, notamment du sang de cordon.

6. La méthode de l'une quelconque des revendications 1 à 5, **caractérisée en ce que** la quantité de PlGF est déterminée en mesurant la quantité d'acide nucléique PlGF.

7. La méthode de l'une quelconque des revendications 1 à 6, **caractérisée en ce que** la quantité de PlGF est mesurée par une méthode sélectionnée parmi le Northern blot, le Southern blot, la PCR, la RT-PCR, la RT-PCR quantitative, le SAGE et ses dérivés, les puces d'acides nucléiques, notamment les puces à ADNc, les puces à oligonucléotides et les puces à ARNm, les puces à tissu et le RNA-Seq.

8. La méthode de l'une quelconque des revendications 1 à 7, **caractérisée en ce que** la quantité de PlGF est déterminée en mesurant la quantité du polypeptide.

9. La méthode de la revendication 8, **caractérisée en ce que** la quantité de PlGF est mesurée par une méthode sélectionnée parmi l'immunohistologie, l'immunoprécipitation, le western blot, le dot blot, l'ELISA ou l'ELISPOT, l'ECLIA, les puces à protéines, les puces à anticorps, ou les puces à tissu couplées à l'immunohistochimie, les techniques de FRET ou de BRET, les méthodes de microscopie ou d'histochimie, dont notamment les méthodes de microscopie confocale et de microscopie électronique, les méthodes basées sur l'utilisation d'une ou plusieurs longueurs d'onde d'excitation et d'une méthode optique adaptée, comme une méthode électrochimique (les techniques de voltammétrie et d'ampérometrie), le microscope à force atomique, et les méthodes de radiofréquence, comme la spectroscopie résonance multipolaire, confocale et non-confocale, détection de fluorescence, luminescence, chemiluminescence, absorbance, réflectance, transmittance, et biréfringence ou index de réfraction (notamment par résonance des plasmons de surface, par ellipsometry ou par méthode de miroir résonnant), cytométrie de flux, imagerie par résonance radioisotopique ou magnétique, analyse par électrophorèse en gel de polyacrylamide (SDS-PAGE); par spectrophotométrie HPLC-Mass, par chromatographie liquide/spectrophotométrie de masse/spectrométrie de masse (LC-MS/MS).

10. La méthode de l'une quelconque des revendications 8 ou 9, **caractérisée en ce que** la quantité de PlGF est déterminée par une méthode choisie parmi l'immunoprécipitation, l'immunohistologie, le western blot, le dot blot, l'ELISA ou l'ELISPOT, l'ECLIA, les puces à protéines, les puces à anticorps, ou les puces à tissu couplées à l'immunohistochimie.

11. La méthode de l'une quelconque des revendications 8 à 10, **caractérisée en ce que** la quantité de PlGF est déterminée par western blot ou par ELISA.

12. La méthode de l'une quelconque des revendications 1 à 11, **caractérisée en ce que** la quantité de PlGF est normalisée par rapport à un marqueur témoin.

13. La méthode de la revendication 12, **caractérisée en ce que** le marqueur témoin est un gène sélectionné dans le groupe constitué de B2M, TFRC, YWHAZ, RPLO, 18S, GUSB, UBC, TBP, GAPDH, PPIA, POLR2A, ACTB, PGK1, HPRT1, IPO8 et HMBS, ou un polypeptide choisi parmi le produit desdits gènes.

## Patentansprüche

1. *In-vitro*-Verfahren zur Diagnose von fetaler Alkoholspektrumstörung (FASD) bei einem Subjekt, umfassend Schritte des:
a) Messens der Menge an plazentarem Wachstumsfaktor (PlGF) in einer biologischen Probe des Subjekts und;
b) Vergleichens der PIGF-Menge aus dem Schritt a) mit einer Referenz, und
c) Bestimmens einer FASD bei dem Subjekt.

2. Verfahren nach Ansprüche 1, **dadurch gekennzeichnet, dass** die Referenz ein Maß der PIGF-Menge bei einem gesunden Individuum ist.

3. Verfahren nach einem der Ansprüche 1 oder 2, **dadurch gekennzeichnet, dass** eine PIGF-Menge aus dem Schritt a), die kleiner ist als die Referenz, darauf hinweist, dass das Subjekt an FASD leidet.

4. Verfahren nach einem der Ansprüche 1 bis 3, **dadurch gekennzeichnet, dass** eine PIGF-Menge aus dem Schritt a), die kleiner ist als die Referenz, auf eine zerebrovaskuläre Störung bei dem Subjekt hinweist.

5. Verfahren nach einem der Ansprüche 1 bis 4, **dadurch gekennzeichnet, dass** die biologische Probe aus der Plazenta, insbesondere dem Nabelschnurblut stammt.

6. Verfahren nach einem der Ansprüche 1 bis 5, **dadurch gekennzeichnet, dass** die PIGF-Menge durch Messen der Menge an PIGF-Nukleinsäure bestimmt wird.

7. Verfahren nach einem der Ansprüche 1 bis 6, **dadurch gekennzeichnet, dass** die PIGF-Menge mittels eines Verfahrens gemessen wird, das ausgewählt ist aus Northern Blot, Southern Blot, PCR, RT-PCR, quantitativer RT-PRC, SAGE und ihren Abwandlungen, den Nukleinsäure-Chips, insbesondere den cDNA-Chips, den Oligonukleotid-Chips und den mRNA-Chips, den Gewebe-Chips und RNA-Seq.

8. Verfahren nach einem der Ansprüche 1 bis 7, **dadurch gekennzeichnet, dass** die PIGF-Menge durch Messen der Menge des Polypeptids bestimmt wird.

9. Verfahren nach Anspruch 8, **dadurch gekennzeichnet, dass** die PIGF-Menge mittels eines Verfahrens gemessen wird, das ausgewählt ist aus Immunhistologie, Immunpräzipitation, Western Blot, Dot Blot, ELISA oder ELISPOT, ECLIA, den Protein-Chips, den Antikörper-Chips, oder den an Immunhistochemie gekoppelten Gewebe-Chips, den FRET- oder BRET-Techniken, den Mikroskopie- oder Histochemie-Verfahren, darunter insbesondere den Konfokalmikroskopie- und Elektronenmikroskopie-Verfahren, den Verfahren, die auf der Verwendung von einer oder mehrerer Anregungswellenlängen und einem abgestimmten optischen Verfahren basieren, wie etwa einem elektrochemischen Verfahren (den Voltammetrie- und Amperometrieverfahren), dem Atomkraftmikroskop, und den Radiofrequenzverfahren, wie der multipolaren, konfokalen oder nicht-konfokalen Resonanzspektroskopie, Fluoreszenz-, Lumineszenz-, Chemilumineszenz-, Absorptions-, Reflexions-, Transmissions- und Doppelbrechungs- oder Brechungsindex-Detektion (insbesondere mittels Oberflächenplasmonenresonanz, mittels Ellipsometrie oder mittels Resonatorspiegelverfahren), Durchflusszytometrie, Radioisotop- oder Magnetresonanzbildgebung, Analyse mittels Polyacrylamidgelelektrophorese (SDS-PAGE); mittels HPLC-Massenspektrophotometrie, mittels Flüssigchromatographie /Massenspektrophotometrie / Massenspektrometrie (LC-MS/MS).

10. Verfahren nach einem der Ansprüche 8 oder 9, **dadurch gekennzeichnet, dass** die PIGF-Menge mittels eines Verfahrens bestimmt wird, das ausgewählt ist aus Immunpräzipitation, Immunhistologie, Western Blot, Dot Blot, ELISA oder ELISPOT, ECLIA, den Protein-Chips, den Antikörper-Chips, oder den an Immunhistochemie gekoppelten Gewebe-Chips.

11. Verfahren nach einem der Ansprüche 8 bis 10, **dadurch gekennzeichnet, dass** die PIGF-Menge mittels Western Blot oder mittels ELISA bestimmt wird.

12. Verfahren nach einem der Ansprüche 1 bis 11, **dadurch gekennzeichnet, dass** die PIGF-Menge in Bezug auf einen Marker normalisiert wird.

13. Verfahren nach Anspruch 12, **dadurch gekennzeichnet, dass** der Marker ein Gen ist, ausgewählt aus der Gruppe bestehend aus B2M, TFRC, YWHAZ, RPLO, 18S, GUSB, UBC, TBP, GAPDH, PPIA, POLR2A, ACTB, PGK1, HPRT1, IPO8 und HMBS, oder ein Polypeptid, das aus dem Produkt der Gene ausgewählt ist.

## Claims

1. An *in vitro* method for the diagnosis of foetal alcohol spectrum disorders (FASDs) in a subject comprising the following steps:
a) measuring the amount of placental growth factor (PlGF) in a biological sample from said subject and;
b) comparing the amount of PlGF from step a) with a reference, and
c) establishing an FASD in said subject.

2. The method of claim 1, **characterised in that** the reference is a measurement of the amount of PlGF in a healthy individual.

3. The method of any one of claims 1 or 2, **characterised in that** an amount of PlGF from step a) lower than the reference indicates than the subject suffers from an FASD.

4. The method of any one of claims 1 to 3, **characterised in that** an amount of PlGF from step a) lower than the reference indicates a brain vascular disorganisation in the subject.

5. The method of any one of claims 1 to 4, **characterised in that** said biological sample derives from the placenta, notably from cord blood.

6. The method of any one of claims 1 to 5, **characterised in that** the amount of PlGF is determined by measuring the amount of PlGF nucleic acid.

7. The method of any one of claims 1 to 6, **characterised in that** the amount of PlGF is measured by a method selected from Northern blot, Southern blot, PCR, RT-PCR, quantitative RT-PCR, SAGE and derivatives thereof, nucleic acid arrays, notably cDNA arrays, oligonucleotide arrays and mRNA arrays, tissue arrays and RNA-Seq.

8. The method of any one of claims 1 to 7, **characterised in that** the amount of PlGF is determined by measuring the amount of the polypeptide.

9. The method of claim 8, **characterised in that** the amount of PlGF is measured by a method selected from immunohistology, immunoprecipitation, Western blot, dot blot, ELISA or ELISPOT, ECLIA, protein arrays, antibody arrays, or tissue arrays coupled with immunohistochemistry, FRET or BRET techniques, microscopy or histochemistry methods, notably including confocal microscopy and electron microscopy methods, methods based on the use one or more excitation wavelengths and a suitable optical method, such as an electrochemical method (voltammetry and amperometry techniques), atomic force microscopy, and radio frequency methods, such as multipolar resonance spectroscopy, confocal and non-confocal, detection of fluorescence, luminescence, chemiluminescence, absorbance, reflectance, transmittance, and birefringence or refractive index (notably by surface plasmon resonance, by ellipsometry or by a resonant mirror method), flow cytometry, radioisotope or magnetic resonance imaging, analysis by polyacrylamide gel electrophoresis (SDS-PAGE), HPLC-mass spectrophotometry and liquid chromatography-mass spectrophotometry/mass spectrometry (LC-MS/MS).

10. The method of any one of claims 8 or 9, **characterised in that** the amount of PlGF is determined by a method selected from immunoprecipitation, immunohistology, Western blot, dot blot, ELISA or ELISPOT, ECLIA, protein arrays, antibody arrays, or tissue arrays coupled with immunohistochemistry.

11. The method of any one of claims 8 to 10, **characterised in that** the amount of PlGF is determined by Western blot or by ELISA.

12. The method of any one of claims 1 to 11, **characterised in that** the amount of PlGF is normalised relative to a control marker.

13. The method of claim 12, **characterised in that** the control marker is a gene selected from the group consisting of B2M, TFRC, YWHAZ, RPLO, 18S, GUSB, UBC, TBP, GAPDH, PPIA, POLR2A, ACTB, PGK1, HPRT1, IPO8 and HMBS, or a polypeptide selected from the products of said genes.
